# FASCICULE DE BREVET EUROPEEN

(11) **EP 1 552 816 B1**
(45) Date de publication et mention de la délivrance du brevet: **08.11.2006**
(21) Numéro de dépôt: 04292777.2
(22) Date de dépôt: 25.11.2004
(51) Int. Cl.: A61K 8/49, A61K 8/89, A61Q 19/08

(54) **Composition cosmétique comprenant un organopolysiloxane élastomère et un composé amino-sulfonique**
Kosmetische Zusammensetzung enthaltend ein Organopolysiloxanelastomer und eine Aminosulfonverbindung.
Cosmetic composition comprising an organosiloxane elastomer and a amino-sulfonic compound.

(30) Priorité: 06.01.2004 FR 0450017
(43) Date de publication de la demande: 13.07.2005
(73) Titulaire: L'ORÉAL, 75008 Paris (FR)
(72) Inventeur: Cotton, Thierry, 06000 Nice (FR)
(74) Mandataire: Kromer, Christophe

(56) Documents cités:
- WO-A-02/39975
- WO-A-99/27791
- US-A1- 2003 157 088

## Description

La présente invention se rapporte à une composition renfermant, dans un milieu physiologiquement acceptable, au moins un organopolysiloxane élastomère et au moins un composé amino-sulfonique donné.

Elle se rapporte également à un procédé cosmétique de soin de la peau, destiné notamment à atténuer les irrégularités visibles ou tactiles de la surface de la peau, en particulier à atténuer les rides et ridules et/ou les taches cutanées et/ou lisser la peau et/ou unifier le teint, comprenant l'application topique sur la peau de la composition précitée.

La peau humaine est constituée de deux compartiments à savoir un compartiment superficiel, l'épiderme, et un compartiment profond, le derme.

L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau.

Au cours du processus dit de kératinisation, les kératinocytes situés dans la couche basale de l'épiderme se multiplient et croissent, poussant ainsi les cellules épidermiques plus anciennes vers le haut et vers la surface de l'épiderme. Lors de ce déplacement, ces cellules s'aplatissent et se différencient pour former de la kératine. Les cellules mortes superficielles résultant de ce processus de kératinisation (cornéocytes) constituent la couche cornée de l'épiderme où elles sont séparées par des couches lipidiques et reliées entre elles par des liaisons protéiniques (cornéosomes). Ces cellules mortes sont progressivement éliminées de la surface de la peau et remplacées par de nouvelles cellules kératinisées.

Dans la peau jeune et saine, la desquamation de la peau qui se produit ainsi est caractérisée par l'élimination de cellules individuelles ou de petits amas cellulaires. Au contraire, avec l'âge ou dans le cas de certaines pathologies, la desquamation peut être altérée, en ce sens qu'un excès de matière kératinique se forme à la surface de la peau, résultant soit en une élimination du stratum corneum sous forme de squames (vieillissement cutané, peau sèche), soit en une obstruction des follicules sébacés (acné).

On connaît dans l'art antérieur divers agents destinés à lutter contre le vieillissement cutané, particulièrement en favorisant la desquamation, c'est-à-dire l'élimination des cellules « mortes » situées à la surface de la couche cornée de l'épiderme. Cette propriété "desquamante" est aussi appelée, souvent à tort, propriété kératolytique.

Ainsi, le brevet US 4,603,146 décrit l'emploi d'acide rétinoïque et de ses dérivés dans des compositions cosmétiques, en vue de lutter contre le vieillissement cutané.

Par ailleurs, de nombreux brevets et publications (voir par exemple la demande EP-A-413 528) ainsi que de nombreuses compositions cosmétiques du commerce enseignent l'emploi des α-hydroxyacides comme l'acide lactique, l'acide glycolique ou encore l'acide citrique pour traiter le vieillissement cutané.

On connaît enfin les β-hydroxyacides et plus spécialement l'acide salicylique ainsi que ses dérivés pour leur propriétés desquamantes (voir les documents WO-A-93/10756 et US 4,767,750).

Bien que leur efficacité soit satisfaisante, ces composés desquamants de l'art antérieur peuvent générer des phénomènes d'irritation ou d'inconfort, en particulier chez les sujets à peau sensible. D'autres desquamants ont donc été proposés, notamment par la Demanderesse dans la demande EP-1 337 233. Il s'agit d'une famille de dérivés amino sulfoniques qui ont la capacité de dégrader les cornéodesmosines (protéines constitutives des cornéosomes) en favorisant l'activité de protéases (de type Chymotrypsine-like et Trypsine-like en particulier), tout en étant bien tolérés.

En raison de leur activité biologique, ces composés n'ont pas un effet immédiat et le bénéfice visible sur la peau du renouvellement épidermique qu'ils entraînent n'est obtenu qu'après quelques jours voire quelques semaines d'utilisation.

Or, la Demanderesse a maintenant découvert qu'en associant ces dérivés amino sulfoniques avec un organopolysiloxane élastomère, il était possible d'améliorer significativement la qualité de surface de la peau dès l'application et de façon prolongée dans le temps.

La présente invention a donc pour objet une composition comprenant, dans un milieu physiologiquement acceptable, au moins un organopolysiloxane élastomère et au moins un composé amino-sulfonique choisi parmi les composés répondant à la formule (I) suivante: 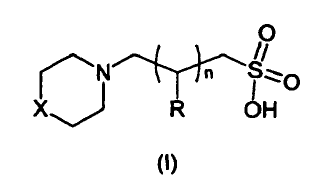 dans laquelle :
- R désigne un atome d'hydrogène ou un groupement choisi parmi -OH et -NH₂,
- X désigne:
   - un atome d'oxygène,
   - un groupement 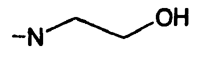
   - un groupement 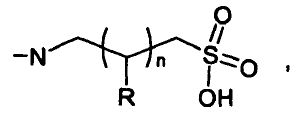
- n est égal à 0, 1, 2 ou 3,
et leurs sels physiologiquement acceptables.

L'invention étend sa portée aux isomères optiques et/ou géométriques des composés de formule (I), seuls ou en mélange en toutes proportions.

Parmi les composés de formule (I) utilisés préférentiellement selon l'invention on peut citer:
- l'acide 4-(2-hydroxyethyl)piperazine-1-ethanesulfonique qui répond à la formule suivante: 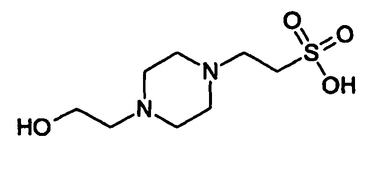
- l'acide 4-(2-hydroxyethyl)piperazine-1-(2-hydroxypropanesulfonique) qui répond à la formule suivante: 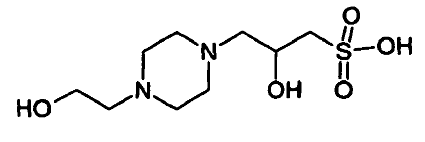
- l'acide 4-(2-hydroxyethyl)piperazine-1-propanesulfonique qui répond à la formule suivante: 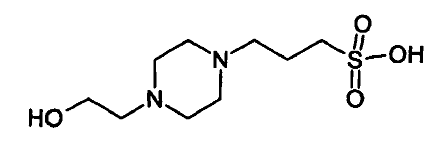
- l'acide 3-morpholinopropanesulfonique qui répond à la formule suivante: 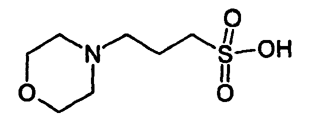
- l'acide 2-morpholinoethanesulfonique qui répond à la formule suivante: 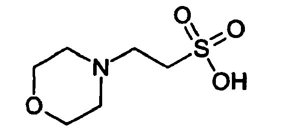
- l'acide piperazine-1,4-bis-(2-ethanesulfonique) qui répond à la formule suivante: 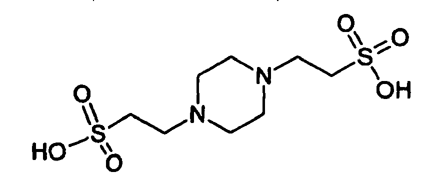
- l'acide piperazine-1,4-bis(2-hydroxypropane sulfonique) qui répond à la formule suivante:

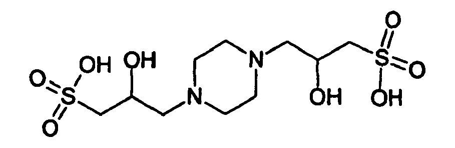

Parmi ces composés, on préfère tout particulièrement l'acide 4-(2-hydroxyethyl)piperazine-1-ethanesulfonique ou HEPES, commercialisé notamment par la société APPLICHEM.

La quantité de composé amino-sulfonique de formule (I) utilisable selon l'invention peut représenter de 0,001 à 20%, de préférence de 0,01 à 10% et, mieux, de 0,1 à 5% du poids total de la composition.

La composition de l'invention contient au moins un organopolysiloxane élastomère, de préférence au moins partiellement réticulé. On entend par « élastomère » un matériau solide souple, déformable ayant des propriétés viscoélastiques et notamment la consistance d'une éponge ou d'une sphère souple. Son module d'élasticité est tel que ce matériau résiste à la déformation et possède une capacité limitée à l'extension et à la contraction. Ce matériau est capable de retrouver sa forme originelle suite à un étirement. Cet élastomère est formé de chaînes polymériques de haut poids moléculaire dont la mobilité est limitée par un réseau uniforme de points de réticulation.

Les organopolysiloxanès élastomères utilisés dans la composition selon l'invention sont de préférence partiellement ou totalement réticulés. Ils se présentent sous forme de particules. En particulier, les particules d'organopolysiloxane élastomère ont une taille allant de 0,1 à 500 µm, de préférence de 3 à 200 µm et mieux de 3 à 50 µm. Ces particules peuvent avoir toute forme et par exemple être sphériques, plates ou amorphes.

L'organopolysiloxane réticulé élastomère peut être obtenu par réaction d'addition réticulation d'un diorganopolysiloxane contenant au moins un atome d'hydrogène lié à un atome de silicium et d'un diorganopolysiloxane ayant au moins deux groupements à insaturation éthylénique liés à des atomes de silicium distincts, notamment en présence de catalyseur platine ; ou par réaction de condensation réticulation déhydrogénation entre un diorganopolysiloxane à terminaisons hydroxyle et un diorganopolysiloxane contenant au moins un atome d'hydrogène lié à un atome de silicium, notamment en présence d'un organoétain ; ou par réaction de condensation réticulation d'un diorganopolysiloxane à terminaisons hydroxyle et d'un organopolysilane hydrolysable ; ou par réticulation thermique d'organopolysiloxane, notamment en présence de catalyseur organopéroxyde ; ou par réticulation d'organopolysiloxane par radiations de haute énergie telles que rayons gamma, rayons ultraviolet, faisceau électronique.

De préférence, l'organopolysiloxane réticulé élastomère est obtenu par réaction d'addition réticulation (A) d'un diorganopolysiloxane contenant au moins un atome d'hydrogène lié à un atome de silicium, et (B) d'un diorganopolysiloxane ayant au moins deux groupements à insaturation éthylénique liés chacun à un atome de silicium distinct, notamment en présence (C) de catalyseur platine, comme par exemple décrit dans la demande EP-A-295886.

Le composé (A) est en particulier un organopolysiloxane ayant au moins 2 atomes d'hydrogène liés à des atomes de silicium distincts dans chaque molécule.

Le composé (A) peut présenter toute structure moléculaire, notamment une structure chaîne linéaire ou chaîne ramifiée ou une structure cyclique. Le composé (A) peut avoir une viscosité à 25 °C allant de 1 à 50 000 centistokes, notamment pour être bien miscible avec le composé (B).

Les groupes organiques liés aux atomes de silicium du composé (A) peuvent être des groupes alkyles tels que méthyl, éthyl, propyl, butyl, octyl ; des groupes alkyles substitués tels que 2-phényléthyl, 2-phénylpropyl, 3,3,3-trifluoropropyl ; des groupes aryles tels que phényl, tolyl, xylyl ; des groupes aryles substitués tels que phényléthyl ; et des groupes hydrocarbonés monovalents substitués tels qu'un groupe époxy, un groupe ester carboxylate, ou un groupe mercapto.

Le composé (A) peut ainsi être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane.

Le composé (B) est avantageusement un diorganopolysiloxane ayant au moins deux groupes alkényles inférieurs (par exemple en C₂-C₄) ; le groupe alkényle inférieur peut être choisi parmi les groupes vinyl, allyl, et propényl. Ces groupements alkényles inférieurs peuvent être situés en toute position de la molécule organopolysiloxane mais sont de préférence situés aux extrémités de la molécule organopolysiloxane. L'organopolysiloxane (B) peut avoir une structure à chaîne ramifiée, à chaîne linéaire, cyclique ou en réseau mais la structure en chaîne linéaire est préférée. Le composé (B) peut avoir une viscosité allant de l'état liquide à l'état de gomme. De préférence, le composé (B) a une viscosité d'au moins 100 centistokes à 25 °C.

Outre les groupes alkényle précités, les autres groupes organiques liés aux atomes de silicium dans le composé (B) peuvent être des groupes alkyles tels que méthyl, éthyl, propyl, butyl ou octyl ; des groupes alkyles substitués tels que 2-phényléthyl, 2-phénylpropyl ou 3,3,3-trifluoropropyl ; des groupes aryles tels que phényl, tolyl ou xylyl ; des groupes aryles substitués tels que phényléthyl ; et des groupes hydrocarbonés monovalents substitués tels qu'un groupe époxy, un groupe ester carboxylate, ou un groupe mercapto.

Les organopolysiloxanes (B) peuvent être choisis parmi les méthylvinylpolysiloxanes, les copolymères méthylvinylsiloxane-diméthylsiloxane, les diméthylpolysiloxanes à terminaisons diméthylvinylsiloxy, les copolymères diméthylsiloxane-méthylphénylsiloxane à terminaisons diméthylvinylsiloxy, les copolymères diméthylsiloxane-diphénylsiloxane-méthylvinylsiloxane à terminaisons diméthylvinylsiloxy, les copolymères diméthylsiloxane-méthylvinylsiloxane à terminaisons triméthylsiloxy, les copolymères diméthylsiloxane-méthylphénylsiloxane-méthylvinylsiloxane à terminaisons triméthylsiloxy, les méthyl(3,3,3-trifluoropropyl)polysiloxane à terminaisons diméthylvinylsiloxy, et les copolymères diméthylsiloxane-méthyl(3,3,3-trifluoropropyl)siloxane à terminaisons diméthylvinylsiloxy.

En particulier, l'organopolysiloxane élastomère peut être obtenu par réaction de diméthylpolysiloxane à terminaisons diméthylvinylsiloxy et de méthylhydrogénopolysiloxane à terminaisons triméthylsiloxy, en présence de catalyseur platine.

Avantageusement, la somme du nombre de groupements éthyléniques par molécule du composé (B) et du nombre d'atomes d'hydrogène liés à des atomes de silicium par molécule du composé (A) est d'au moins 5.

Il est avantageux que le composé (A) soit ajouté en une quantité telle que le rapport moléculaire entre la quantité totale d'atomes d'hydrogène liés à des atomes de silicium dans le composé (A) et la quantité totale de tous les groupements à insaturation éthylénique dans le composé (B) soit compris dans la gamme de 1,5/1 à 20/1.

Le composé (C) est le catalyseur de la réaction de réticulation et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support.

Le catalyseur (C) est de préférence ajouté à raison de 0,1 à 1000 parties en poids, mieux de 1 à 100 parties en poids, en tant que métal platine propre pour 1000 parties en poids de la quantité totale des composés (A) et (B).

L'élastomère obtenu peut être un élastomère non-émulsionnant ou un élastomère émulsionnant.

Le terme "non émulsionnant" défini des élastomères organopolysiloxane ne contenant pas de motifs polyoxyalkylène. Le terme "émulsionnant" signifie des élastomères organopoysiloxanes réticulés ayant au moins un motif polyoxyalkylène, notamment polyoxyéthylène ou polyoxypropylène.

Les particules d'organopolysiloxane réticulé élastomère peuvent être véhiculées sous forme de gel constitué d'un organopolysiloxane élastomérique inclus dans au moins une huile hydrocarbonée et/ou une huile siliconée. Dans ces gels, les particules d'organopolysiloxane sont souvent des particules non-sphériques.

Les particules d'organopolysiloxane réticulé élastomère peuvent également se présenter sous forme de poudre, notamment sous forme de poudre sphérique.

Des élastomères non-émulsionnants sont notamment décrits dans les brevets US4970252, US4987169, US 5412004, US5654362, US5760116, dans la demande JP-A-61-194009.

Comme élastomères non-émulsionnants, on peut utiliser ceux vendus sous les dénominations "KSG-6", "KSG-15", "KSG-16", "KSG-18", "KSG-31", "KSG-32", "KSG-33", "KSG-41", "KSG-42", "KSG-43", "KSG-44" par la société Shin Etsu, "DC 9040", "DC9041", "DC 9509", "DC9505", "DC 9506" par la société Dow Corning, "GRANSIL" par la société Grant Industries, "SFE 839" par la société General Electric.

Avantageusement, les élastomères émulsionnants comprennent les élastomères modifiés polyoxyalkylène formé à partir de composés divinyliques, en particulier des polysiloxanes ayant au moins deux groupes vinyliques, réagissant avec des liaisons Si-H d'un polysiloxane. Des élastomères émulsionnants sont notamment décrits dans les brevets US5236986, US5412004, US5837793, US5811487.

Comme élastomères émulsionnants, on peut utiliser ceux commercialisés sous les dénominations "KSG-21", "KSG-20", "KSG-30", "X-226146" par la société Shin Etsu, et "DC9010", "DC9011" par la société Dow Corning.

Les particules d'organopolysiloxane réticulé élastomère peuvent se présenter également sous forme de poudre d'organopolysiloxane réticulé élastomère enrobée de résine de silicone, notamment de résine silsesquioxane, comme décrit par exemple dans le brevet US5538793. De tels élastomère sont vendus sous les dénomination "KSP-100", "KSP-101", "KSP-102", "KSP-103", KSP-104", "KSP-105" par la société Shin Etsu.

D'autres organopolysiloxanes réticulés élastomères sous forme de poudres peuvent être des poudres de silicone hybride fonctionnalisée par des groupes fluoroalkyle, notamment vendues sous la dénomination "KSP-200" par la société Shin Etsu ; ou des poudres de silicones hybrides fonctionnalisées par des groupes phényl, notamment vendues sous la dénomination "KSP-300" par la société Shin Etsu.

De manière préférée, l'organopolysiloxane élastomère utilisé dans la composition de l'invention est susceptible d'être obtenu par hydrosilylation de polydiméthylsiloxanes à groupements vinyles terminaux, comprenant de 35 à 45 unités diméthylsiloxane, par des poly méthylhydrogénosiloxane diméthylsiloxane, comprenant deux unités méthylhydrogénosiloxanes et de 25 à 35, et plus préférentiellement 30 unités diméthylsiloxanes (KSG-6 de SHIN-ETSU).

La composition selon l'invention renferme par exemple de 0,1 à 30% en poids, mieux, de 0,5 à 10% en poids et, encore mieux, de 1 à 5% en poids d'organopolysiloxane élastomère.

La composition selon l'invention est généralement adaptée à une application topique sur la peau et comprend donc généralement un milieu physiologiquement acceptable, c'est-à-dire compatible avec la peau et/ou ses phanères. Ainsi, la composition selon l'invention a de préférence un pH inférieur à 8, mieux, inférieur ou égal à 7 et, encore mieux, compris entre 6 et 7.

La composition selon invention peut se présenter sous toutes les formes galéniques classiquement utilisées pour une application topique et notamment sous forme de dispersions du type lotion ou gel, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore d'émulsions multiples (E/H/E ou H/E/H), de microémulsions, de dispersions vésiculaires de type ionique et/ou non ionique, ou de dispersions cire/phase aqueuse. Ces compositions sont préparées selon les méthodes usuelles.

Selon un mode préféré de réalisation de l'invention, la composition se présente sous forme d'une émulsion H/E.

Comme huiles utilisables dans la composition selon l'invention, on peut citer :
- les huiles hydrocarbonées d'origine animale, telles que le perhydrosqualène ;
- les huiles hydrocarbonées d'origine végétale, telles que les huiles végétales, en particulier la fraction liquide du beurre de karité;
- les esters et les éthers de synthèse, notamment d'acides gras, comme par exemple les esters du pentaérythritol tels que le tétraéthylhexanoate de pentaérythrityle ;
- les hydrocarbures linéaires ou ramifiés, d'origine minérale ou synthétique, tels que le polyisobutène hydrogéné ;
- les alcools gras ayant de 8 à 26 atomes de carbone, comme l'alcool cétylique, l'alcool stéarylique et leur mélange, ou l'octyldodécanol ;
- les huiles de silicone comme les polyméthylsiloxanes (PDMS) volatiles ou non à chaîne siliconée linéaire ou cyclique ; et
- leurs mélanges.

Lorsqu'elle est présente, la phase grasse de la composition selon l'invention peut comprendre, outre des huiles, d'autres corps gras tels que : les acides gras comportant de 8 à 30 atomes de carbone ; les cires ; et les gommes telles que les gommes de silicone (diméthiconol).

Ces corps gras peuvent être choisis de manière variée par l'homme du métier afin de préparer une composition ayant les propriétés, par exemple de consistance ou de texture, souhaitées.

Cette composition peut en outre contenir divers adjuvants couramment utilisés dans le domaine cosmétique, tels que des émulsionnants dont le stéarate de glycéryle et les stéarates de poly(éthylène glycol) ; des charges, notamment à effet soft-focus telles que les dispersions colloïdales de silice, ou encore des copolymères blocs tels que décrits dans US-6,013,682 ; des conservateurs ; des séquestrants ; des colorants ; des parfums ; et des épaississants et gélifiants, en particulier les polyacrylamides et les copolymères acryliques. Les quantités de ces différents adjuvants et leur nature seront choisis de manière à ne pas nuire aux propriétés de la composition selon l'invention.

Selon une forme d'exécution préférée, la composition selon l'invention comprend en outre au moins un hydroxyacide et/ou au moins un polyholoside hétérogène

Comme hydroxyacides, on peut citer les α-hydroxyacides, tels que les acides citrique, lactique, glycolique, tartrique, mandélique et/ou malique, et les β-hydroxyacides, tels que l'acide salicylique et ses dérivés, en particulier l'acide n-octanoyl-5-salicylique.

On utilise de préférence selon l'invention une combinaison d'acide n-octanoyl-5-salicylique et d'acide glycolique.

Les hydroxyacides peuvent représenter de 0,001 à 20%, de préférence de 0,01 à 10% et, mieux, de 0,1 à 1% du poids total de la composition.

Par "polyholoside hétérogène", on entend selon la présente invention des polymères constitués de l'association d'oses différents ou d'oses ayant la même formule chimique brute mais de configuration géométrique différente (isomères D et L par exemple). Ces polymères se distinguent à la fois des polyhétérosides, qui sont constitués d'un ou plusieurs oses et d'une partie non glucidique, et des polyholosides homogènes, qui résultent de l'association d'un même ose. Ainsi, lorsqu'il est présent dans la composition selon l'invention, le polyholoside hétérogène est constitué uniquement de sucres et résulte de l'association d'au moins deux oses différents.

Les polyholosides utilisés dans la composition selon invention peuvent être constitués de 2 à 10 oses, composés couramment appelés oligoholosides, ou de plus de 10 oses, composés couramment appelés polyholosides.

Les oses présents dans le polyholoside selon l'invention peuvent être choisis parmi tous les oses envisageables, d'origine naturelle ou synthétique, et notamment tels que :
- les aldoses comme
   - les pentoses : ribose, arabinose, xylose ou apiose, par exemple,
   - les hexoses : glucose, fucose, mannose ou galactose, par exemple,
- les cétoses tels que le fructose,
- les désoxyoses, tels que le rhamnose, le digitoxose, le cymarose ou l'oléandrose,
- les dérivés d'ose tels que les acides uroniques comme les acides mannuronique, guluronique, galacturonique ou glycuronique, ou encore les itols comme le mannitol ou le sorbitol.

Dans le cadre de la présente invention, on peut utiliser un polyholoside hétérogène seul, ou un mélange de polyholosides hétérogènes.

De préférence, le polyholoside hétérogène comprend au moins un motif fucose, qui peut être présent en une quantité de 10-90% en poids, de préférence 15-35% en poids, par rapport au poids de matière sèche de polyholoside.

En particulier, le polyholoside selon l'invention peut comprendre des motifs fucose, galactose et acide galacturonique, et, par exemple peut comprendre un enchaînement linéaire de α-L-Fucose, de α-D-Galactose et d'acide galacturonique. Un tel polyholoside est notamment disponible auprès de la société SOLABIA sous la dénomination commerciale Fucogel 1000 PP^{®} .

Les polyholosides selon l'invention sont de préférence introduits dans la composition sous forme de solution aqueuse qui peut comprendre 0,1 à 5% en poids de polyholoside.

Le polyholoside peut être présent dans la composition finale en une quantité de 0,001 à 50% en poids, de préférence de 0,01 à 10% en poids et, mieux, de 0,01 à 0,1% en poids.

Pour renforcer les effets de la composition selon l'invention, celle-ci peut renfermer par ailleurs au moins un composé choisi parmi : les agents hydratants tels que les céramides, l'acide hyaluronique et en particulier la glycérine ; les agents dépigmentants tels que la vitamine C et ses dérivés ; les agents stimulant la différenciation des kératinocytes ; et les agents anti-pollution et/ou anti-radicalaires, notamment le tocophérol et ses esters.

La composition selon l'invention peut en outre contenir au moins un filtre UVA et/ou UVB. Les filtres solaires peuvent être choisis parmi les filtres organiques, les filtres inorganiques et leurs mélanges.

Les filtres organiques plus particulièrement préférés sont choisis parmi les composés suivants : l'éthylhexyl salicylate, l'éthylhexyl méthoxycinnamate, l'octocrylène, l'acide phénylbenzimidazole sulfonique, la benzophénone-3, la benzophénone-4, la benzophénone-5, le 4-méthylbenzylidène camphre, l'acide téréphthalylidène dicamphre sulfonique, la 2,4,6-tris-(4'-amino benzalmalonate de diisobutyle)-s-triazine, l'anisotriazine, l'éthylhexyl triazone, la diéthylhexyl butamido triazone, le méthylène bis-benzotriazolyl tétraméthylbutylphénol, le drometrizole trisiloxane, les dérivés de 4,4-diarylbutadiène, et leurs mélanges.

Les filtres inorganiques sont choisis parmi des pigments ou bien encore des nanopigments (taille moyenne des particules primaires : généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium.

La composition selon l'invention permet d'estomper sans inconfort les irrégularités visibles et tactiles de la surface cutanée.

L'invention a donc aussi pour objet un procédé cosmétique de soin de la peau, notamment pour atténuer les irrégularités visibles ou tactiles de la surface de la peau, en particulier pour atténuer les rides et ridules et/ou les taches cutanées et/ou lisser la peau et/ou unifier le teint, comprenant l'application topique sur la peau de la composition précitée.

L'invention sera maintenant illustrée par les exemples non limitatifs suivants. Dans ces exemples, les quantités sont indiquées en pourcentage pondéral.

### EXEMPLES

### Exemple 1 : Crème pour peaux sèches

On prépare une émulsion H/E ayant la composition ci-dessous, de façon classique pour l'homme du métier.

| | | |
|---|---|---|
| Acide glycolique | | 0,05 % |
| Acide n-octanoyl-5-salicylique | | 0,20 % |
| Polyholoside hétérogène à 1 % dans l'eau (Fucogel 1000 PP de SOLABIA) | | 5,00 % |
| Acide hydroxyéthyl pipérazine éthane sulfonique | | 1,00 % |
| Alcool | | 2,00 % |
| Glycérine | | 3,00 % |
| Tocophérol à 50% dans l'huile de soja | | 0,10 % |
| Stéarate de glycéryle | | 0,50 % |
| Elastomère de silicone dans l'huile | | |
| (KSG-6 de SHIN-ETSU) | | 2,60% |
| EDTA disodique | | 0,05 % |
| Huiles | | 25,00 % |
| Charges | | 2,50% |
| Polymères épaississants | | 2,00 % |
| Conservateurs | | qs |
| Neutralisant | qs | pH = 6,5 |
| Parfum | | qs |
| Eau | qsp | 100,00% |

Cette composition peut être appliquée matin et/ou soir sur le visage pour hydrater et lisser la peau.

### Exemple 2 : Crème pour peaux normales à mixtes

On prépare une émulsion H/E ayant la composition ci-dessous, de façon classique pour l'homme du métier.

| | | |
|---|---|---|
| Acide lactique | | 0,05 % |
| Acide salicylique | | 0,30 % |
| Polyholoside hétérogène à 1% dans l'eau (Fucogel 1000 PP de SOLABIA) | | 5,00 % |
| Acide hydroxyéthyl pipérazine éthane sulfonique | | 1,00% |
| Alcool | | 4,00 % |
| Glycérine | | 3,00 % |
| Tocophérol à 50% dans l'huile de soja | | 0,10% |
| Stéarate de glycéryle | | 0,50 % |
| Elastomère de silicone dans l'huile (KSG-6 de SHIN-ETSU) | | 2,60 % |
| EDTA disodique | | 0,05 % |
| Huiles | | 20,00 % |
| Charges | | 2,50 % |
| Polymères épaississants | | 2,00 % |
| Conservateurs | | qs |
| Neutralisant | qs | pH = 6,5 |
| Parfum | | qs |
| Eau | qsp | 100,00% |

Cette composition peut être appliquée matin et/ou soir sur le visage pour lisser et affiner le grain de peau et unifier le teint.

## Revendications

1. Composition renfermant, dans un milieu physiologiquement acceptable, au moins un organopolysiloxane élastomère et au moins un composé amino-sulfonique choisi parmi les composés répondant à la formule (I) suivante: 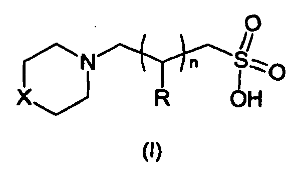 dans laquelle :
- R désigne un atome d'hydrogène ou un groupement choisi parmi -OH et -NH₂,
- X désigne:
- un atome d'oxygène,
- un groupement 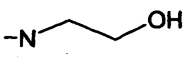
- un groupement 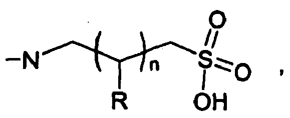
- n est égal à 0, 1, 2 ou 3,
et leurs sels physiologiquement acceptables.

2. Composition selon la revendication 1, **caractérisée en ce que** l'organopolysiloxane est partiellement ou totalement réticulé.

3. Composition selon la revendication 2, **caractérisée en ce que** l'organopolysiloxane réticulé élastomère est obtenu par réaction d'addition réticulation (A) d'un diorganopolysiloxane contenant au moins un atome d'hydrogène lié à un atome de silicium, et (B) d'un diorganopolysiloxane ayant au moins deux groupements à insaturation éthylénique liés chacun à un atome de silicium distinct.

4. Composition selon la revendication 3, **caractérisée en ce que** le composé (A) est choisi parmi les méthylhydrogénopolysiloxanes à terminaisons triméthylsiloxy, les copolymères diméthylsiloxane-méthylhydrogénosiloxane à terminaisons triméthylsiloxy, et les copolymères cycliques diméthylsiloxane-méthylhydrogénosiloxane.

5. Composition selon la revendication 3 ou 4, **caractérisée en ce que** le organopolysiloxanes (B) est choisi parmi les méthylvinylpolysiloxanes, les copolymères méthylvinylsiloxane-diméthylsiloxane, les diméthylpolysiloxanes à terminaisons diméthylvinylsiloxy, les copolymères diméthylsiloxane-méthylphénylsiloxane à terminaisons diméthylvinylsiloxy, les copolymères diméthylsiloxane-diphénylsiloxane-méthylvinylsiloxane à terminaisons diméthylvinylsiloxy, les copolymères diméthylsiloxane-méthylvinylsiloxane à terminaisons triméthylsiloxy, les copolymères diméthylsiloxane-méthylphénylsiloxane-méthylvinylsiloxane à terminaisons triméthylsiloxy, les méthyl(3,3,3-trifluoropropyl)polysiloxane à terminaisons diméthylvinylsiloxy, et les copolymères diméthylsiloxane-méthyl(3,3,3-trifluoropropyl)siloxane à terminaisons diméthylvinylsiloxy.

6. Composition selon l'une quelconque des revendications 2 à 5, **caractérisée en ce que** l'organopolysiloxane élastomère peut être obtenu par réaction de diméthylpolysiloxane à terminaisons diméthylvinylsiloxy et de méthylhydrogénopolysiloxane à terminaisons triméthylsiloxy, en présence de catalyseur platine.

7. Composition selon la revendication 6, **caractérisée en ce que** l'organopolysiloxane élastomère est susceptible d'être obtenu par hydrosilylation de polydiméthylsiloxanes à groupements vinyles terminaux, comprenant de 35 à 45 unités diméthylsiloxane, par des poly méthylhydrogénosiloxanes diméthylsiloxanes, comprenant deux unités méthylhydrogénosiloxanes et de 25 à 35 unités diméthylsiloxanes.

8. Composition selon la revendication 7, **caractérisée en ce que** le poly méthylhydrogénosiloxane diméthylsiloxane comprend 30 unités diméthylsiloxanes.

9. Composition selon l'une quelconque des revendications 1 à 8, **caractérisée en ce que** le composé amino-sulfonique est l'acide 4-(2-hydroxyethyl)piperazine-1-ethanesulfonique.

10. Composition selon l'une quelconque des revendications 1 à 9, **caractérisée en ce qu'**elle renferme de 1 à 5% en poids d'organopolysiloxane.

11. Composition selon l'une quelconque des revendications 1 à 10, **caractérisée en ce qu'**elle renferme de 0,1 à 5% en poids de composé de formule (I).

12. Composition selon l'une quelconque des revendications 1 à 11, **caractérisée en ce qu'**elle renferme en outre au moins un hydroxyacide et/ou un polyholoside hétérogène.

13. Composition selon la revendication 12, **caractérisée en ce que** l'hydroxyacide est un α-hydroxyacide.

14. Composition selon la revendication 13, **caractérisée en ce que** l'α-hydroxyacide est choisi parmi les acides citrique, lactique, glycolique, tartrique, mandélique et/ou malique.

15. Composition selon la revendication12, **caractérisée en ce que** l'hydroxyacide est un β-hydroxyacide.

16. Composition selon la revendication 15, **caractérisée en ce que** le β-hydroxyacide est choisi parmi l'acide salicylique et l'acide n-octanoyl-5-salicylique.

17. Composition selon la revendication 12, **caractérisée en ce qu'**elle renferme une combinaison d'acide n-octanoyl-5-salicylique et d'acide glycolique.

18. Composition selon l'une quelconque des revendications 12 à 17, **caractérisée en ce que** le polyholoside comprend un enchaînement linéaire de α-L-Fucose, de α-D-Galactose et d'acide galacturonique.

19. Composition selon l'une quelconque des revendications 1 à 18, **caractérisée en ce qu'**elle se présente sous forme d'une émulsion H/E.

20. Procédé cosmétique de soin de la peau comprenant l'application topique sur la peau de la composition selon l'une quelconque des revendications 1 à 19.

21. Procédé cosmétique pour atténuer les irrégularités visibles ou tactiles de la surface de la peau, en particulier pour atténuer les rides et ridules et/ou les taches cutanées et/ou lisser la peau et/ou unifier le teint, comprenant l'application topique sur la peau de la composition selon l'une quelconque des revendications 1 à 19.

## Claims

1. Composition including, in a physiologically acceptable medium, at least one organopolysiloxane elastomer and at least one aminosulphonic compound chosen from the compounds corresponding to the following formula (I): 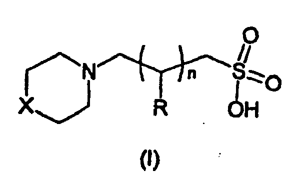 in which:
- R denotes a hydrogen atom or a group chosen from -OH and -NH₂,
- X denotes:
- an oxygen atom,
- a 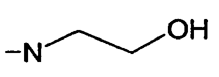 group,
- a 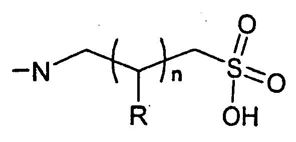 group,
- n is equal to 0, 1, 2 or 3,
and their physiologically acceptable salts.

2. Composition according to Claim 1, **characterized in that** the organopolysiloxane is partially or completely crosslinked.

3. Composition according to Claim 2, **characterized in that** the crosslinked organopolysiloxane elastomer is obtained by a crosslinking addition reaction (A) of a diorganopolysiloxane comprising at least one hydrogen atom bonded to a silicon atom and (B) of a diorganopolysiloxane having at least two groups comprising ethylenic unsaturation each bonded to a separate silicon atom.

4. Composition according to Claim 3, **characterized in that** the compound (A) is chosen from methylhydropolysiloxanes comprising trimethylsiloxy endings, dimethylsiloxane/methylhydrosiloxane copolymers comprising trimethylsiloxy endings and dimethylsiloxane/methylhydrosiloxane cyclic copolymers.

5. Composition according to Claim 3 or 4, **characterized in that** the organopolysiloxane (B) is chosen from methylvinylpolysiloxanes, methylvinylsiloxane/dimethylsiloxane copolymers, dimethylpolysiloxanes comprising dimethylvinylsiloxy endings, dimethylsiloxane/methylphenylsiloxane copolymers comprising dimethylvinylsiloxy endings, dimethylsiloxane/diphenylsiloxane/methylvinylsiloxane copolymers comprising dimethylvinylsiloxy endings, dimethylsiloxane/methylvinylsiloxane copolymers comprising trimethylsiloxy endings, dimethylsiloxane/methylphenylsiloxane/methylvinylsiloxane copolymers comprising trimethylsiloxy endings, methyl(3,3,3-trifluoropropyl)polysiloxanes comprising dimethylvinylsiloxy endings and dimethylsiloxane/methyl(3,3,3-trifluoropropyl)siloxane copolymers comprising dimethylvinylsiloxy endings.

6. Composition according to any one of Claims 2 to 5, **characterized in that** the organopolysiloxane elastomer can be obtained by reaction of a dimethylpolysiloxane comprising dimethylvinylsiloxy endings and of a methylhydropolysiloxane comprising trimethylsiloxy endings in the presence of a platinum catalyst.

7. Composition according to Claim 6, **characterized in that** the organopolysiloxane elastomer is capable of being obtained by hydrosilylation of polydimethylsiloxanes comprising end vinyl groups, comprising from 35 to 45 dimethylsiloxane units, by polymethylhydrosiloxane/dimethylsiloxanes comprising two methylhydrosiloxane units and from 25 to 35 dimethylsiloxane units.

8. Composition according to Claim 7, **characterized in that** the polymethylhydrosiloxane/dimethylsiloxane comprises 30 dimethylsiloxane units.

9. Composition according to any one of Claims 1 to 8, **characterized in that** the aminosulphonic compound is 4-(2-hydroxyethyl)piperazine-1-ethanesulphonic acid.

10. Composition according to any one of Claims 1 to 9, **characterized in that** it includes from 1 to 5% by weight of organopolysiloxane.

11. Composition according to any one of Claims 1 to 10, **characterized in that** it includes from 0.1 to 5% by weight of compound of formula (I).

12. Composition according to any one of Claims 1 to 11, **characterized in that** it additionally includes at least one hydroxy acid and/or one heterogeneous polysaccharide.

13. Composition according to Claim 12, **characterized in that** the hydroxy acid is an α-hydroxy acid.

14. Composition according to Claim 13, **characterized in that** the α-hydroxy acid is chosen from citric acid, lactic acid, glycolic acid, tartaric acid, mandelic acid and/or malic acid.

15. Composition according to Claim 12, **characterized in that** the hydroxy acid is a β-hydroxy acid.

16. Composition according to Claim 15, **characterized in that** the β-hydroxy acid is chosen from salicylic acid and 5-(n-octanoyl)salicylic acid.

17. Composition according to Claim 12, **characterized in that** it includes a combination of 5-(n-octanoyl)salicylic acid and of glycolic acid.

18. Composition according to any one of Claims 12 to 17, **characterized in that** the polysaccharide comprises a linear sequence of α-L-fucose, of α-D-galactose and of galacturonic acid.

19. Composition according to any one of Claims 1 to 18, **characterized in that** it is provided in the form of an O/W emulsion.

20. Cosmetic process for caring for the skin, comprising the topical application to the skin of the composition according to any one of Claims 1 to 19.

21. Cosmetic process for toning down visible or tactile irregularities of the surface of the skin, in particular for toning down wrinkles and fine lines and/or cutaneous blemishes, and/or smoothing the skin and/or unifying the complexion, comprising the topical application to the skin of the composition according to any one of Claims 1 to 19.

## Patentansprüche

1. Zusammensetzung, die in einem physiologisch akzeptablen Medium mindestens ein elastomeres Organopolysiloxan und mindestens eine Aminosulfonsäureverbindung enthält, die unter den Verbindungen der folgenden Formel (I), worin bedeuten: 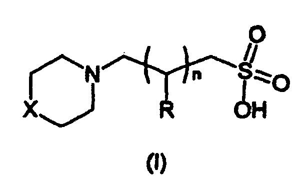 worin bedeuten:
- R ein Wasserstoffatom oder eine Gruppe, die unter -OH und -NH₂ ausgewählt ist,
- X:
- ein Sauerstoffatom,
- eine Gruppe 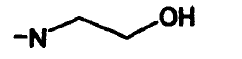
- eine Gruppe 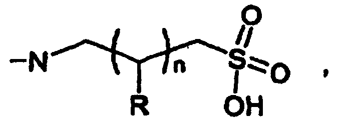
- n 0, 1, 2 oder 3,
und deren physiologisch akzeptablen Salzen ausgewählt ist.

2. Zusammensetzung nach Anspruch 1, **dadurch gekennzeichnet, dass** das Organopolysiloxan ganz oder teilweise vernetzt ist.

3. Zusammensetzung nach Anspruch 2, **dadurch gekennzeichnet, dass** das vernetzte elastomere Organopolysiloxan durch eine vernetzende Addition (A) eines Diorganopolysiloxans, das mindestens ein an ein Siliciumatom gebundenes Wasserstoffatom aufweist, und (B) eines Diorganopolysiloxans gebildet wird, das mindestens zwei Gruppen mit ethylenisch ungesättigter Bindung aufweist, die jeweils an ein unterschiedliches Siliciumatom gebunden sind.

4. Zusammensetzung nach Anspruch 3, **dadurch gekennzeichnet, dass** die Verbindung (A) unter den Methylhydrogenopolysiloxanen mit endständigen Trimethylsiloxygruppen, Dimethylsiloxan/Methylhydrogenosiloxan-Copolymeren mit endständigen Trimethylsiloxygruppen und cyclischen Dimethylsiloxan/Methylhydrogenosiloxan-Copolymeren ausgewählt ist.

5. Zusammensetzung nach Anspruch 3 oder 4, **dadurch gekennzeichnet, dass** das Organopolysiloxan (B) unter den Methylvinylpolysiloxanen, Methylvinylsiloxan/Dimethylsiloxan-Copolymeren, Dimethylpolysiloxanen mit endständigen Dimethylvinylsiloxygruppen, Dimethylsiloxan/Methylphenylsiloxan-Copolymeren mit endständigen Dimethylvinylsiloxygruppen, Dimethylsiloxan/Diphenylsiloxan/Methylvinylsiloxan-Copolymeren mit endständigen Dimethylvinylsiloxygruppen, Dimethylsiloxan/Methylvinylsiloxan-Copolymeren mit endständigen Trimethylsiloxygruppen, Dimethylsiloxan/ Methylphenylsiloxan/ Methylvinylsiloxan-Copolymeren mit endständigen Trimethylsiloxygruppen, Methyl(3,3,3-trifluorpropyl)polysiloxanen mit endständigen Dimethylvinylsiloxygruppen und Dimethylsiloxan/Methyl (3,3,3-trifluorpropyl)siloxan-Copolymeren mit endständigen Dimethylvinylsiloxygruppen ausgewählt ist.

6. Zusammensetzung nach einem der Ansprüche 2 bis 5, **dadurch gekennzeichnet, dass** das elastomere Organopolysiloxan durch Umsetzung eines Dimethylpolysiloxans mit endständigen Dimethylvinylsiloxygruppen und eines Methylhydrogenopolysiloxans mit endständigen Trimethylsiloxygruppen in Gegenwart eines Platinkatalysators erhalten werden kann.

7. Zusammensetzung nach Anspruch 6, **dadurch gekennzeichnet, dass** das elastomere Organopolysiloxan durch Hydrosilylierung von Polydimethylsiloxanen mit endständigen Vinylgruppen, die 35 bis 45 Dimethylsiloxaneinheiten enthalten, mit Poly(methylhydrogenosiloxanen-dimethylsiloxanen), die zwei Methylhydrogenosiloxaneinheiten und 25 bis 35 Dimethylsiloxaneinheiten enthalten, erhältlich ist.

8. Zusammensetzung nach Anspruch 7, **dadurch gekennzeichnet, dass** das Poly(methylhydrogenosiloxan-dimethylsiloxan) 30 Dimethylsiloxaneinheiten aufweist.

9. Zusammensetzung nach einem der Ansprüche 1 bis 8, **dadurch gekennzeichnet, dass** es sich bei der Aminosulfonsäureverbindung um die 4-(2-Hydroxyethyl)piperazin-1-ethansulfonsäure handelt.

10. Zusammensetzung nach einem der Ansprüche 1 bis 9, **dadurch gekennzeichnet, dass** sie 1 bis 5 Gew.-% Organopolysiloxan enthält.

11. Zusammensetzung nach einem der Ansprüche 1 bis 10, **dadurch gekennzeichnet, dass** sie 0,1 bis 5 Gew.-% der Verbindung der Formel (I) enthält.

12. Zusammensetzung nach einem der Ansprüche 1 bis 11, **dadurch gekennzeichnet, dass** sie ferner mindestens eine Hydroxysäure und/oder ein heterogenes Polyholosid enthält.

13. Zusammensetzung nach Anspruch 12, **dadurch gekennzeichnet, dass** die Hydroxysäure eine α-Hydroxysäure ist.

14. Zusammensetzung nach Anspruch 13, **dadurch gekennzeichnet, dass** die α-Hydroxysäure unter Citronensäure, Milchsäure, Glycolsäure, Weinsäure, Mandelsäure und/ oder Äpfelsäure ausgewählt ist.

15. Zusammensetzung nach Anspruch 12, **dadurch gekennzeichnet, dass** die Hydroxysäure eine β-Hydroxysäure ist.

16. Zusammensetzung nach Anspruch 15, **dadurch gekennzeichnet, dass** die β-Hydroxysäure unter Salicylsäure und 5-n-Octanoylsalicylsäure ausgewählt ist.

17. Zusammensetzung nach Anspruch 12, **dadurch gekennzeichnet, dass** sie 5-n-Octanoylsalicylsäure in Kombination mit Glycolsäure enthält.

18. Zusammensetzung nach einem der Ansprüche 12 bis 17, **dadurch gekennzeichnet, dass** das Polyholosid eine lineare Verknüpfung von α-L-Fucose, α-D-Galactose und Galacturonsäure enthält.

19. Zusammensetzung nach einem der Ansprüche 1 bis 18, **dadurch gekennzeichnet, dass** sie in Form einer O/W-Emulsion vorliegt.

20. Kosmetisches Verfahren zur Pflege der Haut, das das topische Auftragen der Zusammensetzung nach einem der Ansprüche 1 bis 19 auf die Haut umfasst.

21. Kosmetisches Verfahren, um die sichtbaren oder fühlbaren Unregelmäßigkeiten der Hautoberfläche abzumildern, insbesondere Falten und Fältchen und/oder die Flecken der Haut abzuschwächen und/oder die Haut zu glätten und/oder den Teint zu vereinheitlichen, das das topische Auftragen der Zusammensetzung nach einem der Ansprüche 1 bis 19 auf die Haut umfasst.
